(19) **Europäisches Patentamt**
**European Patent Office**
**Office européen des brevets**

(11) **EP 1 509 201 B1**

(12) **EUROPÄISCHE PATENTSCHRIFT**

(45) Veröffentlichungstag und Bekanntmachung des Hinweises auf die Patenterteilung:
**25.03.2009 Patentblatt 2009/13**

(21) Anmeldenummer: **03735367.9**

(22) Anmeldetag: **08.05.2003**

(51) Int Cl.:
***A61K 9/70*** (2006.01)　　　***A61K 31/465*** (2006.01)

(86) Internationale Anmeldenummer:
**PCT/EP2003/004816**

(87) Internationale Veröffentlichungsnummer:
**WO 2003/101421 (11.12.2003 Gazette 2003/50)**

(54) **WIRKSTOFFHALTIGE FILMFÖRMIGE ZUBEREITUNGEN MIT VERBESSERTER CHEMISCHER STABILITÄT, UND VERFAHREN ZU DEREN HERSTELLUNG**

FILM-SHAPED PREPARATIONS WITH IMPROVED CHEMICAL STABILITY CONTAINING ACTIVE SUBSTANCES AND METHOD FOR THE PRODUCTION THEREOF

PREPARATIONS CONTENANT DES SUBSTANCES ACTIVES, SOUS FORME DE FILM, A STABILITE CHIMIQUE AMELIOREE ET PROCEDES DE PRODUCTION DESDITES PREPARATIONS

(84) Benannte Vertragsstaaten:
**AT BE BG CH CY CZ DE DK EE ES FI FR GB GR HU IE IT LI LU MC NL PT RO SE SI SK TR**

(30) Priorität: **04.06.2002 DE 10224612**

(43) Veröffentlichungstag der Anmeldung:
**02.03.2005 Patentblatt 2005/09**

(73) Patentinhaber: **LTS LOHMANN Therapie-Systeme AG**
**56626 Andernach (DE)**

(72) Erfinder:
• **VON FALKENHAUSEN, Christian**
**53340 Meckenheim (DE)**
• **MÜLLER, Walter**
**56626 Andernach (DE)**

• **KRUMME, Markus**
**Randolph, NJ 07869 (US)**

(74) Vertreter: **Flaccus, Rolf-Dieter**
**Patentanwalt**
**Bussardweg 10**
**50389 Wesseling (DE)**

(56) Entgegenhaltungen:
**EP-A- 0 380 367　　　EP-A- 0 539 215**
**WO-A-02/066016　　　US-B1- 6 183 775**

• **DUNNETT P C ET AL: "STUDY OF THE FATE OF BRONOPOL AND THE EFFECTS OF ANTIOXIDANTS ON N NITROSAMINE FORMATION IN SHAMPOOS AND SKIN CREAMS" INTERNATIONAL JOURNAL OF COSMETIC SCIENCE, Bd. 6, Nr. 5, 1984, Seiten 241-248, XP009016766 ISSN: 0142-5463**

**Beschreibung**

Die Erfindung betrifft wirkstoffhaltige filmförmige zubereitungen, die zur Applikation in der Mundhöhle und zur transmukosalen Verabreichung von Wirkstoffen verwendet werden können, und die sich durch eine verbesserte chemische Stabilität auszeichnen.
Die Erfindung erstreckt sich ferner auf Herstellungsverfahren, mittels welcher wirkstoffhaltige Filme der genannten Art erhalten werden können.

Wirkstoffhaltige Filme weisen im allgemeinen eine Matrix auf, die ein oder mehrere Polymere als Grundsubstanzen enthält. In dieser Polymermatrix ist mindestens ein Wirkstoff, z. B. ein Arzneistoff, in gelöster oder dispergierter Form enthalten. Häufig werden der Matrix verschiedenartige weitere Hilfsstoffe oder Zusatzstoffe zugesetzt beispielsweise zur Einstellung verschiedener physikalischer oder pharmazeutischer Parameter. Derartige filmförmige Zubereitungen sind in der Lage, die enthaltenen Wirkstoffe am Applikationsort, beispielsweise in der Mundhöhle, freizusetzen, so daß sie vom Organismus resorbiert werden können.

Viele pharmazeutische Wirkstoffe sind chemisch instabil und können in Abbauprodukte zerfallen, beispielsweise durch längere Lagerung. Durch diese Abbaureaktionen wird die anfänglich vorhandene Wirkstoffmenge verringert, und die gebildeten Abbauprodukte können toxikologisch bedenklich sein. Infolge dieser Abbaureaktionen wird deshalb die Haltbarkeit der Arzneiformen verringert.
Für diese Abbaureaktionen sind einerseits bestimmte physikalische Parameter verantwortlich (z. B. Temperatur, Lichteinwirkung), andererseits aber auch die chemische Zusammensetzung der pharmazeutischen Zusammensetzung.

Unter den Abbaureaktionen sind vor allem oxidative Reaktionen zu erwähnen; diese treten besonders stark ausgeprägt in Gegenwart von aktivem Sauerstoff, z. B. in Gegenwart von Peroxiden, auf. Dabei handelt es sich um Autoxidationsreaktionen, die als Kettenreaktionen ablaufen. Derartige Autoxidationsprozesse und die zugrundeliegenden Reaktionsmechanismen sind dem Fachmann grundsätzlich bekannt. Bei filmförmigen Zubereitungen ist in besonderem Maße mit dem Auftreten unerwünschter oxidativer Prozesse zu rechnen, da diese eine relative große Oberfläche aufweisen, die dem Angriff des Luftsauerstoffs ausgesetzt sein kann.

Um derartige Abbaureaktionen zu unterdrücken, werden derartige Arzneiformen üblicherweise unter Sauerstoffausschluß, z. B. unter einer Stickstoffatmosphäre, hergestellt und verpackt, oder es werden Antioxidantien verwendet.
Es hat sich jedoch gezeigt, daß trotz dieser Vorsichtsmaßnahmen bei längerer Lagerung von filmförmigen wirkstoffhaltigen Zubereitungen eine mehr oder weniger starke Abnahme des Wirkstoffgehalts eintritt, insbesondere dann, wenn es sich um oxidationsempfindliche Wirkstoffe handelt.

Die der vorliegenden Erfindung zugrunde liegende Aufgabe bestand deshalb darin, filmförmige wirkstoffhaltige Zubereitungen der im Oberbegriff des Anspruchs 1 genannten Art anzugeben, die eine verbesserte Wirkstoff-Stabilität aufweisen. Ferner bestand die Aufgabe darin, Verfahren aufzuzeigen, welche die Herstellung solcher Zubereitungen ermöglichen.

Überraschenderweise wird diese Aufgabe durch Zubereitungen und Herstellungsverfahren gemäß den vorliegenden Ansprüchen gelöst.

Erfindungsgemäß läßt sich die Wirkstoffstabilität bei den genannten filmförmigen Zubereitungen dadurch verbessern, indem die Peroxidzahl der Zubereitung bei der Herstellung auf einen Wert von höchstens 40, vorzugsweise höchstens 15, insbesondere von höchstens 5 eingestellt wird, dadurch gekennzeichnet, dass die genannte Peroxidzahl erzielt wird durch Verwendung mindestens eines Antioxidans ausgewählt aus der Gruppe umfassend Natriumsulfit, Natriummetabisulfit, Thioglycerol, Thioglycolsäure, Propylgallat, Octylgallat, Butylhydroxyanisol und Butylhydroxytoluol, jeweils in einer Konzentration von 0,001 bis 5 Gew.-%.

EP0380367 A und EP0539215 A erwähnen weder die im vorliegenden Anspruch 1 genannten Antioxidans, noch den Begriff "Peroxidzahl".

Die Peroxidzahl ist ein Maß für den Gehalt an Peroxiden; sie gibt die Menge an Milli-Äquivalenten aktiven Sauerstoffs pro kg einer Substanz an. Aufgrund der Begrenzung der Peroxidzahl auf einen Höchstwert von 40, vorzugsweise von 15 und insbesondere von 5, sind die erfindungsgemäßen filmförmigen Zubereitungen im wesentlichen frei von aktivem Sauerstoff.

Unter "aktivem Sauerstoff" wird Sauerstoff verstanden, der eine Oxidationsstufe aufweist, die größer als -2 ist. Insbesondere umfaßt dieser Begriff molekularen Sauerstoff sowie Peroxide der allgemeinen Struktur R-O-O-R', worin R und R' H-Atome sind, oder R ein Alkylrest und R' ein H-Atom ist, oder R und R' Alkylreste sind, wobei R und R' identisch oder verschieden sein können.

Es hat sich gezeigt, daß eine akzeptable, über Monate andauernde Lagerstabilität nur erreicht werden kann, wenn der relative Anteil des aktiven Sauerstoffs den Wert von 2 Gew.-% nicht übersteigt. Beispielsweise kann eine wirkstoffhaltige filmförmige Zubereitung ("wafer") 200 mg eines wirkstoffs mit einem Molekulargewicht von 250 Dalton enthalten, welches 0,8 mMol Wirkstoff entspricht. Der Anteil des aktiven Sauerstoffs darf in diesem Fall nicht mehr als 2 % dieses Wertes betragen, d. h. ein Gehalt von 0,016 mMol aktiven Sauerstoffs darf nicht überschritten werden. Dieser Wert entspricht einer Peroxidzahl von ca. 30. Bei dem genannten Wafer kann es sich beispielsweise um einen lutschbaren Wafer mit einem Flächengewicht von 500 g/m$^2$ und einer Wirkstoffbeladung von 40 % handeln, mit einer Flächenausdehnung von 10 cm$^2$.

Im Falle von dünneren, schnell freisetzenden Wafern mit einer niedrigeren Wirkstoffbeladung ergibt sich eine entsprechend niedrigere Obergrenze für Gehalt an aktivem Sauerstoff. wenn der vorstehend erwähnte Wirkstoff (Molekulargewicht 250 Dalton) in einer Menge von 14 mg in einem Wafer enthalten ist, entspricht dies 0,056 mMol Wirkstoff, und folglich darf der Gehalt an aktivem Sauerstoff den Wert von 0,001 mMol (entsprechend 2 %) nicht überschreiten. Dies entspricht einer Peroxidzahl von ca. 14. Der genannte Wafer kann beispielsweise ein Flächengewicht von 70 g/m$^2$ und eine Wirkstoffbeladung von 20 Gew.-% aufweisen, wobei das Eigengewicht eines einzelnen Systems (Wafer) 70 mg beträgt.

Für die Bestimmung der Peroxidzahl gibt es verschiedene bekannte Methoden.

(A) Am gebräuchlichsten ist die Umsetzung einer definierten Menge der zu testenden Substanz in einer Chloroform/Eisessig-Lösung mit einem Überschuß an Iodid-Ionen und anschließender Rücktitration des gebildeten Iods mit Natriumthiosulfat.

(B) Weniger gebräuchlich und auf wässrige Lösungen beschränkt ist die Umsetzung der zu testenden Substanz mit Titan(IV)-Ionen und die photometrische Bestimmung des sich bildenden Peroxokomplexes.

(C) Besonders einfach durchzuführen ist ein semiquantitativer Peroxid-Test mit kommerziell erhältlichen Teststäbchen.

Die Erfindung beruht auf der Erkenntnis, daß die bei der Herstellung der filmförmigen Zubereitungen verwendeten Rohstoffe oder Formulierungsbestandteile im Ausgangszustand häufig relativ hohe Konzentrationen an Hydroperoxiden und Peroxiden aufweisen. Dies trifft häufig auf Polymere, Lösemittel und bestimmte Zusatzstoffe (z. B. Permeations-Enhancer) zu. Begünstigt durch die Gegenwart von Luftsauerstoff und Schwermetall-Verunreinigungen, kommt es zu Radikal-Kettenreaktionen, in deren Verlauf bestimmte Bindungen in Wirkstoffmolekülen angegriffen werden, z. B. C-H-Bindungen in Benzyl- oder Allylstellung, tertiäre C-H-Bindungen und C-H-Bindungen in der Nähe von Ethersauerstoffatomen. Wirkstoffmoleküle, die über solche Gruppen verfügen, sind in besonderem Maße durch Peroxid-Angriff gefährdet.

Durch die erfindungsgemäß vorgeschlagene Verminderung der Peroxidzahl in der Zubereitung läßt sich die durch Radikalkettenreaktionen verursachte Wirkstoffinstabilität wesentlich wirksamer unterdrücken, als dies durch die bekannten Maßnahmen - Zusatz von Antioxidantien, Stickstoff-Atmosphäre - möglich ist. Der Grund hierfür ist vermutlich, daß der Zusatz von Antioxidantien oder Stabilisatoren dann nicht mehr wirksam ist, wenn schon die verwendeten Ausgangsmaterialien bzw. Formulierungsbestandteile relativ hohe Konzentrationen an Peroxiden bzw. Hydroperoxid-Radikalen enthalten.

Grundsätzlich läßt sich die Peroxidzahl der hergestellten filmförmigen Zubereitungen nach den oben beschriebenen Methoden (A) oder (B) bestimmen. Allerdings kann es unter Umständen schwierig sein, eine ausreichende Menge der zu testenden filmförmigen Zubereitung in einer nicht zu großen Menge des angegebenen Lösemittels zu lösen.

Zur Vereinfachung wird deshalb vorzugsweise so vorgegangen, daß der Peroxid-Gehalt eines jeden Formulierungsbestandteils der filmförmigen Zusammensetzung einzeln bestimmt wird (beispielsweise nach einer der oben angegebenen Methoden), und anschließend die Peroxidzahl der Zusammensetzung berechnet wird, wobei die Peroxidzahlen der einzelnen Formulierungsbestandteile entsprechend ihrem prozentualen Anteil an der Zusammensetzung gewichtet und schließlich addiert werden; diese Summe stellt die Gesamt-Peroxidzahl der Zusammensetzung dar. Bei der Berechnung der Gesamt-Peroxidzahl ist auch der Peroxid-Gehalt der bei der Herstellung verwendeten Lösemittel zu berücksichtigen.

Die Berechnung der Gesamt-Peroxidzahl wird durch folgendes Rechenbeispiel veranschaulicht:

Eine filmförmige Zusammensetzung besteht aus drei Formulierungsbestandteilen X, Y und Z, wobei der Anteil von X 70 Gew.-%, der Anteil von Y 20 Gew.-% und der Anteil von Z 10 Gew.-% beträgt. Für Bestandteil X wurde eine Peroxidzahl von 10 bestimmt, im Falle von Y und Z beträgt die Peroxidzahl 15 bzw. 30.

Die Gesamt-Peroxidzahl berechnet sich wie folgt:

```
(10 x 70/100) + (15 x 20/100) + (30 x 10/100) =
= 7 + 3 + 3 = 13.
```

Wie ersichtlich, werden die Peroxidzahlen der einzelnen Bestandteile mit einem Faktor gewichtet, der ihrem prozentualen Anteil an der Zusammensetzung entspricht.

um die Stabilität der in den filmförmigen Zusammensetzungen enthaltenen Wirkstoffe weiter zu verbessern, ist nach einer bevorzugten Ausführungsform vorgesehen, daß die Zusammensetzung mindestens ein Antioxidans enthält. In Betracht kommen insbesondere Antioxidantien aus Gruppe, Natriumsulfit, Natriumdisulfit, Natrium-metabisulfit, Thioglycerol, Thioglykolsäure, Propylgallat, Octylgallat, Butylhydroxyanisol und Butylhydroxytoluol umfaßt.

Die Konzentration dieser Stoffe beträgt vorzugsweise 0,001 bis 5 Gew.-%, besonders bevorzugt 0,01 bis 3 Gew.-%, jeweils bezogen auf die filmförmige Zusammensetzung.

Die erfindungsgemäßen filmförmigen Zusammensetzungen weisen eine Polymermatrix auf, die einschichtig oder auch mehrschichtig sein kann; in jedem Fall ist mindestens eine Schicht wirkstoffhaltig. Die Polymermatrix enthält mindestens ein Polymer, oder ein Polymergemisch, als Grundsubstanz(en). Der Polymer-Anteil beträgt vorzugsweise 10 bis 95 Gew.-%, besonders bevorzugt 25-85 Gew.-%, jeweils bezogen auf die gesamte filmförmige Zusammensetzung. Die Dicke der erfindungsgemäßen wirkstoffhaltigen Filme liegt vorzugsweise im Bereich von 0,01 bis 5 mm, besonders bevorzugt im Bereich von 0,05 bis 1 mm.

Für die Herstellung der Polymermatrix werden insbesondere folgende Polymere bevorzugt: Cellulose-Ether, insbesondere Ethylcellulose, Propylcellulose, Carboxymethylcellulose, Hydroxypropylcellulose, Hydroxypropylmethyl-Cellulose, Gemische von Cellulose-Ethern, sowie Celluloseacetat, Polyvinylalkohole, Polyvinylacetat, Polyvinylpyrrolidone, Polyethylenoxid-Polymere, Polyurethan, Polyacrylsäure, Polyacrylate, Polymethacrylate, Alginate, Pectine, Gelatine, Stärke, und natürliche Gummen.

Eine weitere bevorzugte Ausführungsform der Erfindung sieht vor, daß die Matrix ein oder mehrere Polymer (e) enthält, das/die aus der Gruppe der hydrophilen, wasserlöslichen oder in wässrigen Medien zerfallsfähigen Polymere ausgewählt ist/sind. Auf diese Weise kann die Wirkstoff-Freisetzung aus der Zusammensetzung durch die Löslichkeit bzw. durch die Zerfallsfähigkeit in wässrigen Medien, z. B. in Körperflüssigkeiten, gesteuert werden. Beispielsweise können die Filme wahlweise als schnell oder als langsam freisetzende Systeme formuliert werden.

Als hydrophile, wasserlösliche oder in wässrigen Medien zerfallsfähige Polymere kommen insbesondere in Betracht: Cellulosederivate, insbesondere Hydroxypropylmethylcellulose, Carboxymethylcellulose, Hydroxypropylcellulose und Methylcellulose, sowie Polyvinylalkohol, Polyvinylacetat, Polyvinylpyrrolidon, Polyacrylate, wasserlösliche Polysaccharide, insbesondere Pullulan, Xanthan, Alginate, Dextrane und Pektine, Proteine, vorzugsweise gelbildende Proteine, insbesondere Gelatine.

Ferner ist gemäß einer weiteren bevorzugten Ausführungsform vorgesehen, daß zumindest eine Schicht oder zumindest eine Oberfläche der Zubereitung mukoadhäsive Eigenschaften aufweist. Die mucoadhäsiven Eigenschaften werden im wesentlichen durch die Art des/der matrixbildenden Polymers/Polymere, sowie die relativen Anteile dieser Polymere in der Zubereitung bestimmt. Als matrixbildende Polymere, welche Bestandteile einer erfindungsgemäßen mucoadhäsiven Formulierung sein können, kommen - ohne andere geeignete Rohstoffe auszuschließen - vorzugsweise folgende Polymere in Betracht: Polyvinylalkohol (z. B. Mowiol®); Cellulosederivate wie Hydroxypropylmethylcellulose, Hydroxypropylcellulose, Natrium-Carboxymethylcellulose (z. B . Walocel), methylcellulose, Hydroxyethylcellulose und Hydroxypropylethylcellulose; Stärke und Stärkederivate; Gelatine (verschiedene Typen); Polyvinylpyrrolidon; Gummi arabicum; Pullulan; Acrylate.

Als Wirkstoffe, die in den erfindungsgemäßen Zubereitungen enthalten sind, kommen grundsätzlich alle pharmazeutischen Wirkstoffe in Betracht, sowie alle anderen Wirkstoffe, die geeignet sind, in physiologische Vorgänge bei Mensch oder Tier einzugreifen.

Die erfindungsgemäßen Zubereitungen eignen sich insbesondere zur Verabreichung von Wirkstoffen, die aufgrund ihrer chemischen Struktur in erhöhtem Maße für oxidative Abbaureaktionen empfänglich sind. Hierzu gehören vor allem Wirkstoffmoleküle, die über mindestens eine der folgenden Teilstrukturen verfügen:

- sekundäre oder tertiäre Aminogruppen
- C=C-Doppelbindungen, konjugierte Doppelbindungen
- C-H-Gruppen in Allylstellung
- benzylische C-H-Gruppen
- tertiäre C-H-Gruppen
- Sulfid-, Thioether- oder Sulfoxidgruppen.

Beispiele für solche wirkstoffe sind: Steroide wie 17-beta-Estradiol, heterozyklische Verbindungen wie Dihydropyridine (z. B. Calciumantagonisten vom Dihydropyridin-Typ), Nicotin, (-)-5,6,7,8,-Tetrahydro-6-[propyl[2-(2-thienyl)-ethyl]amino]-1-naphthol, aromatische Verbindungen, insbesondere substituierte aromatische Verbindungen (z. B. Adrenalin, Salizylsäure und -derivate, Phenothiazine); sauerstoffempfindliche Biopolymere, Proteine, oxidationsanfällige Stoffe wie Amine, Hydroxylamine, Alkohole und Aldehyde.

Zur Erzielung bestimmter Wirkungen oder zur Modulation der chemischen oder physikalischen Eigenschaften kann es vorteilhaft sein, wenn die filmförmigen Zubereitungen einen oder mehrere Zusatzstoffe enthalten, ausgewählt aus der Gruppe der Weichmacher, Farbstoffe und Pigmente, Zerfallsförderer, Netzmittel, resorptions- oder permeationsfördernden Substanzen, pH-Regulatoren, Füllstoffe, Geschmacks- und Aromastoffe und Süßstoffe. Hierfür geeignete pharmazeutisch verträgliche Stoffe sind dem Fachmann bekannt. Diese Zusatzstoffe können vorzugsweise in einer Gesamtkonzentration von bis zu 50 Gew.-% enthalten sein, insbesondere in einer Gesamtkonzentration von 1,0 bis 15 Gew.-%.

Als Weichmacher kommen beispielsweise solche aus der Gruppe der Kohlenwasserstoffe, Alkohole (insbesondere höhere Alkohole wie Dodecanol, Undecanol, Octanol), Triglyceride, mehrwertige Alkohole, Carbonsäuren, Derivate von Carbonsäuren, Ether, Ester (z. B. Diethylphthalat, n-Butyladipat, Citronensäure-Ester) und Amine in Betracht.

Zur Verbesserung der physikalischen Eigenschaften kann die wirkstoffmatrix Füllstoffe enthalten, beispielsweise Titandioxid, Zinkoxid, Kreide, Aktivkohle, feinverteiltes Siliciumdioxid oder Maisstärke.

Als Resorptions- oder Permeationsbeschleuniger (Enhancer) eignen sich insbesondere Stoffe, die ausgewählt sind aus der Gruppe, die folgende Stoffe bzw. Stoffklassen umfaßt: gesättigte oder ungesättigte Fettsäuren, Fettsäure-Ester, insbesondere Ester mit Methanol, Ethanol oder isopropanol (z. B. Ölsäureethylester, Ölsäuremethylester, Laurinsäuremethylester, Laurinsäureethylester, Adipinsäuremethylester, Adipinsäureethylester), geradkettige oder verzweigte Fettalkohole bzw. deren Ester, insbesondere Ester mit Essigsäure oder Milchsäure (z. B. Ethyloleat, Ethyllaurat, Ethylpalmitat, Ethyllactat, Propyllactat, Propylpalmitat, Propyllaurat, Propyloleat), mehrwertige aliphatische Alkohole oder Polyethylenglykole, Sorbitanfettsäureester und deren durch Ethoxylierung erhältlichen Derivats, Fettalkoholethoxylate, Polyoxyethylenfettsäureester; Laurinsäurediethanolamid, Ölsäurediethanolamid, Kokosfettsäurediethanolamid, D-alpha-Tocopherol, Laurinsäurehexylester, 2-Octyldodecanol, Dexpanthenol, Isopropylidenglycerol, Transcutol (= Diethylenglycol-monoethylether), DEET (= N,N-Diethyl-m-Toluolamid), Solketal, Ethanol, 1,2-Propandiol oder andere kurzkettige Alkohole (d. h. Alkohole mit bis zu 6 C-Atomen), sowie Menthol und andere ätherische Öle oder Bestandteile ätherischer Öle. Um den Wirkstoff-Flux zu optimieren, können auch Kombinationen zweier oder mehrerer Enhancer eingesetzt werden.

Die erfindungsgemäßen Zubereitungen eignen sich in vorteilhafter Weise zur transmukosalen Verabreichung von Arzneistoffen, beispielsweise über die Mundschleimhaut, aber auch an andere Schleimhautoberflächen des Körpers. Durch die mukoadhäsiven Eigenschaften der wirkstoffhaltigen Filmschicht kann eine kontrollierte Wirkstoffabgabe über einen längeren Zeitraum hinweg erfolgen. Die filmförmigen Zubereitungen können vorzugsweise zur Freisetzung von Wirkstoffen oder anderen Stoffen, z.B. von Geschmacks- oder Aromastoffen, in der Mundhöhle eingesetzt werden. Die erfindungsgemäßen Zubereitungen können ferner auch als orale Darreichungsformen verwendet werden, welche die Freisetzung und/oder Resorption von pharmazeutischen Wirkstoffen im Gastrointestinaltrakt ermöglichten. Beispielsweise kann im Falle von lutschbaren filmförmigen Zubereitungen die beim Lutschen entstehende wirkstoffhaltige Lösung oder Suspension geschluckt und anschließend im Gastrointestinaltrakt resorbiert werden. Als lutschbare wirkstoffhaltige Systeme werden bevorzugt relativ dicke Filme verwendet, vorzugsweise mit einer Dicke von bis zu 5 mm, insbesondere von 0,5 bis 5 mm.

Die Erfindung umfaßt aber auch solche orale filmförmige Arzneiformen, die zum Schlucken bestimmt sind und bei denen die Wirkstofffreisetzung im wesentlichen erst im Gastrointestinaltrakt einsetzt. Dies schließt auch solche filmförmige wirkstoffhaltige System mit ein, die nach oraler Verabreichung zunächst in der Mundhöhle in Bruchstücke zerfallen, die dann geschluckt werden.

Die vorliegende Erfindung betrifft des weiteren Herstellungsverfahren, mittels derer filmförmige Zubereitungen der vorstehend genannten Art erhalten werden können. Erfindungsgemäß erfolgt die Herstellung auf die Weise, daß in einem ersten Schritt die Peroxidzahl eines jeden der für die Herstellung der Zubereitung gemäß Rezeptur vorgesehenen Formulierungsbestandteils (einschließlich der verwendeten Lösemittel) bestimmt wird.

Anschließend werden in einem weiteren Schritt die Formulierungsbestandteile in der Weise ausgewählt, daß die Summe der Peroxidzahlen der einzelnen Formulierungsbestandteile höchstens 40 beträgt, wobei die Peroxidzahl eines jeden Formulierungsbestandteils entsprechend dem prozentualen Anteil dieses Bestandteils an der Zubereitung gewichtet wird.

Aus diesen so ausgewählten Formulierungsbestandteilen wird eine Lösung, Dispersion oder Schmelze hergestellt, welche den/die freizusetzenden Wirkstoff(e) enthält.

Diese Lösung, Dispersion oder Schmelze wird durch Rakel-, Walzenauftrags-, Sprüh- oder Extrusionsverfahren auf eine inerte Unterlage beschichtet und getrocknet oder abkühlen lassen, wodurch eine Filmschicht gebildet wird.

Falls sich bei der Bestimmung der Peroxidzahlen der einzelnen Komponenten herausstellt, daß ein zu hoher Peroxid-Gehalt vorliegt, so kann entweder ein Ersatzstoff für diese Komponente (z. B. ein Rohstoff eines anderen Herstellers) ausgewählt werden, der möglicherweise eine niedrigere Peroxidzahl aufweist, oder der betreffende Formulierungsbestandteil wird einer Behandlung unterworfen, welche geeignet ist, den Peroxid-Gehalt zu vermindern. Hierzu kommt eine Behandlung mit Reduktionsmittel(n) in Betracht, beispielsweise mit einem anorganischen Sulfit oder Hydrogensulfit, vorzugsweise mit Natriumsulfit oder Natriumhydrogensulfit, jeweils in wässriger Lösung (z. B. 5 bis 30 Gew.-%). Der betreffende Formulierungsbestandteil wird dabei in einer alkoholischen Lösung, vorzugsweise in methanolischer oder ethanolischer Lösung, mit der genannten wässrigen Lösung des Reduktionsmittels versetzt. Durch diese Behandlung werden die anwesenden Peroxide in schneller Reaktion problemlos zerstört.

Abhängig von den Lösungseigenschaften des zu behandelnden Bestandteils kann dieser auch in einem wässrigen Lösungsmittel gelöst werden, oder in einem Alkohol-Wasser-Gemisch. Falls es sich bei dem zu behandelnden Formulierungsbestandteil oder Hilfsstoff um eine Flüssigkeit handelt (z. B. Lösemittel), kann die Behandlung in der Weise erfolgen, daß diese Flüssigkeit direkt mit einer wässrigen Lösung des Reduktionsmittels (z. B. Natriumsulfit) versetzt wird.

Die Verwendung von Natriumsulfit bzw. Natriumhydrogensulfit ist besonders vorteilhaft, da es sich hierbei um pharmazeutisch erlaubte Hilfsstoffe handelt, so daß eine spätere Abtrennung nicht erforderlich ist.

Falls es zur Ausfällung von Reaktionsprodukten kommt, können diese durch Zentrifugation, Sedimentation oder Filtration abgetrennt werden.

Nach dieser Behandlung sind die Materialien praktisch frei von Peroxiden und können selbst bei vorheriger erheblichen Belastung bedenkenlos eingesetzt werden. Eine zusätzliche Verbesserung der Stabilität kann durch den Zusatz von Antioxidantien erreicht werden, die die Bildung von neuen Peroxiden während der Lagerung der Systeme unterdrücken bzw. verlangsamen.

Die Erfindung und ihre vorteilhaften Eigenschaften werden durch die nachfolgenden Beispiele näher erläutert.

Es wurden filmförmige Zubereitungen nach folgenden Rezepturen hergestellt:

Beispiel 1: (Nicht die Erfindung)

| Ethanol/ Wasser | |
|---|---|
| Nikotin | 15% |
| HPMC | 79,998% |
| Menthol | 5% |
| Ascorbylpalmitat | 0,002 % |

Beispiel 2:

| Ethanol/ Wasser | |
|---|---|
| Nikotin | 15% |
| HPMC | 79,99% |
| Menthol | 5% |
| Natriumdisulfit | 0,01 % |

Beispiel 3: (Nicht die Erfindung)

| Ethanol/ Wasser | |
|---|---|
| Nikotin | 15% |
| HPMC | 79,95% |
| Menthol | 5% |
| Vitamin E | 0,05 % |

Vergleichsbeispiel:

wie Beispiel 1, jedoch ohne Ascorbylpalmitat.

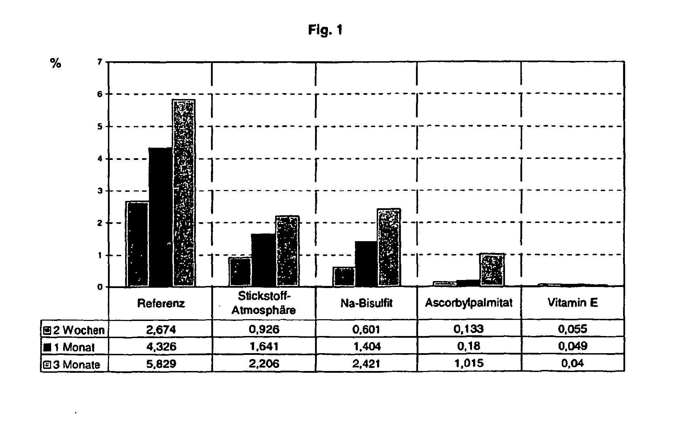

Filmförmige Zubereitungen mit den in den Beispielen angegebenen Zusammensetzungen wurden einem Stabilitäts-Test unterzogen. Hierbei wurden die Filme bei 40 °C und einer relativen Luftfeuchtigkeit von 75 % gelagert und in bestimmten zeitabständen (2 Wochen, 1 Monat, 3 Monate) die durch oxidative Prozesse bedingte Verringerung des Wirkstoffgehalts bestimmt. Die Ergebnisse sind in Fig. 1 dargestellt, wobei die Prozentwerte den Gehalt an Abbauprodukten, bezogen auf den Gehalt an aktiver Substanz (Wirkstoff), angeben.

**Patentansprüche**

1. Filmförmige wirkstoffhaltige Zubereitung zur Applikation in der Mundhöhle oder zur transmukosalen Applikation mit einer Peroxidzahl von höchstens 40, **dadurch gekennzeichnet, dass** die genannte Peroxidzahl erzielt wird durch Verwendung mindestens eines Antioxidans ausgewählt aus der Gruppe umfassend Natriumsulfit, Natriummetabisulfit, Thioglycerol, Thioglycolsäure, Propylgallat, Octylgallat, Buthylhydroxyanisol und Butylhydroxytoluol, jeweils in einer Konzentration von 0,001 bis 5 Gew.%.

2. Zubereitung nach Anspruch 1 **dadurch gekennzeichnet, dass** eine Peroxidzahl von höchstens 15 durch Verwendung mindestens eines der genannten Antioxidantien in einer Konzentration von 0,01 bis 3 Gew.% erzielt wird.

3. Zubereitung nach Anspruch 2, **dadurch gekennzeichnet, dass** eine Peroxidzahl von höchstens 5 erzielt wird.

4. Zubereitung nach einem der vorhergehenden Ansprüche **dadurch gekennzeichnet, dass** sie im wesentlichen frei ist von aktivem Sauerstoff, wobei sich der Begriff "aktiver Sauerstoff" auf molekularen Sauerstoff sowie auf sauerstoffhaltige Verbindungen bezieht, in denen Sauerstoff eine höhere Oxidationsstufe als -2 aufweist, insbesondere Peroxide mit der allgemeinen Struktur R-O-O-R, worin R und R aus der Gruppe ausgewählt sind, die aus Alkylresten und Wasserstoff besteht, und wobei R und R gleich oder verschieden sein können.

5. Zubereitung nach einem der vorangehenden Ansprüche, **dadurch gekennzeichnet, dass** sie eine ein- oder mehrschichtige Polymermatrix aufweist, wobei mindestens eine Schicht wirkstoffhaltig ist.

6. Zubereitung nach Anspruch 5 **dadurch gekennzeichnet, dass** die Matrix ein oder mehrere Polymer(e) enthält, die aus der Gruppe ausgewählt ist/sind, welche Cellulose-Ether, insbesondere Ethylcellulose, Propylcellulose, Carboxymethylcellulose (CMC), Hydroxypropylcellulose (HPC), Hydroxypropylmethylcellulose (HPMC) Gemischen aus Cellulose-Ethern, sowie Celluloseacetat, Polyviinylalkohole, Polyvinylacetat, Polyvinylpyrrolidone, Polyethylenoxid-Polymere, Polyurethan, Polyacrylsäure, Polyacrylate, Polymethacrylate, Alginate, Pectine, Gelatine, Stärke, und natürliche Gummen umfaßt.

7. Zubereitung nach Anspruch 5 **dadurch gekennzeichnet, dass** die Matrix ein oder mehrere Polymer(e) enthält, das/die aus der Gruppe der hydrophilen, wasserlöslichen oder in wäßrigen Medien zerfallsfähigen Polymere ausgewählt ist/sind, vorzugsweise aus der Gruppe, welche Cellulosederivate, insbesondere Hydroxypropylmethylcellulose, Carboxymethylcellulose, Hydroxypropylcellulose und Methylcellulose, sowie Polyvinylalkohol, Polyvinylacetat, Polyvinylpyrrolidon, Polyacrylate, wasserlösliche Polysaccharide, insbesondere Pullulan, Xanthan; Alginate, Dextrane und Pektine, Proteine, vorzugsweise gelbildende Proteine, insbesondere Gelatine umfaßt.

8. Zubereitung nach einem der vorangehenden Ansprüche **dadurch gekennzeichnet, dass** zumindest eine schicht oder zumindest eine Oberfläche der Zubereitung mukoadhäsive Eigenschaften aufweist.

9. Zubereitung nach einem der vorangehenden Ansprüche, **dadurch gekennzeichnet, dass** sie einen oder mehrere Zusatzstoffe enthält, ausgewählt aus der Gruppe der Weichmacher, Farbstoffe und Pigmente, Zerfallsförderer, Netzmittel, resorptions- oder permeationsfördernden Substanzen, pH-Regulatoren, Füllstoffe, Geschmacks- und Aromastoffe und Süßstoffe.

10. Zubereitung nach einem der vorangehenden Ansprüche, **dadurch gekennzeichnet, dass** sie mindestens einen Wirkstoff enthält, der aufgrund seiner chemischen Struktur für einen Angriff durch Peroxidradikale empfänglich ist.

11. Verwendung einer Zubereitung nach einem der vorangehenden Ansprüche zur Herstellung eines Produktes zur transmukosalen Verabreichung von Arzneimittelwirkstoffen, vorzugsweise zur Applikation in der Mundhöhle.

12. Verwendung einer Zubereitung nach einem der vorangehenden Ansprüche zur Herstellung einer Produktes als orale Darreichungsform zur Freisetzung von Wirkstoffen im Gastrointestinaltrakt.

13. Verwendung einer Zubereitung nach einem der vorangehenden Ansprüche zur Herstellung eines Produktes zur Freisetzung von Geschmacks- oder Aromastoffen in der Mundhöhle.

14. Verfahren zur Herstellung einer filmförmigen wirkstoffhaltigen Zubereitung zur Applikation in der Mundhöhle zur transmukosalen Applikation gemäß Anspruch1 **gekennzeichnet durch** folgende Arbeitsschritte:

(a) Bestimmung der Peroxidzahl eines jeden der für die Herstellung oder Zubereitung gemäß Rezeptur vorgesehenen Formulierungsbestandteils;

(b) Auswahl der Formulierungsbestandteile in der Weise, dass die Summe der Peroxidzahlen der einzelnen Formulierungsbestandteile höchstens 40 beträgt, wobei die Peroxidzahl eines jeden Formulierungsbestandteils entsprechend dem prozentualen Anteil dieses Bestandteils an der Zubereitung gewichtet wird;

(c) Herstellung einer Lösung, Dispersion oder Schmelze, welche die ausgewählten Formulierungsbestandteile sowie den/die freizusetzenden Wirkstoff(e) enthält;

(d) Beschichtung dieser Lösung, Dispersion oder Schmelze auf eine inerte Unterlage **durch** Rakel-, Walzenauftrags-, Sprüh- oder Extrusionsverfahren und nachfolgende Trocknung oder Abkühlung, wodurch eine Filmschicht gebildet wird.

**15.** Verfahren nach Anspruch 14 **dadurch gekennzeichnet, dass** die Summe der Peroxidzahlen höchstens 15, vorzugsweise höchstens 5 beträgt.

**16.** Verfahren nach Anspruch 14 oder 15 **dadurch gekennzeichnet, dass** mindestens ein Formulierungsbestandteil im Anschluß an Schritt (a) einer Behandlung mit Reduktionsmittel(n) unterworfen wird, welche(s) geeignet ist/sind, den Peroxid-gehalt zu vermindern.

**17.** Verfahren nach Anspruch 16, **dadurch gekennzeichnet, dass** die genannte Behandlung in der Weise durchgeführt wird, dass der Formulierungsbestandteil in einer alkoholischen Lösung, vorzugsweise in methanolischer oder ethanolischer Lösung, mit der wäßrigen Lösung eines anorganischen Sulfitsalzes oder Hydrogensulfitsalzes versetzt wird.

**Claims**

**1.** Film-like, active substance-containing preparation for application in the oral cavity or for transmucosal application, with a peroxide number of maximally 40, **characterized in that** the above-mentioned peroxide number is obtained through the use of at least one antioxidant selected from the group which comprises sodium sulfite, sodium metabisulfite, thioglycerol, thioglycolic acid, propyl gallate, octyl gallate, butylhydroxyanisol and butylhydroxytoluene, in each case at a concentration of 0,001 to 5%-wt.

**2.** Preparation according to claim 1, **characterized in that** a peroxide number of maximally 15 is obtained through the use of at least one of the mentioned antioxidants at a concentration of 0.01 to 3%-wt.

**3.** Preparation according to claim 2, **characterized in that** a peroxide number of maximally 5 is obtained.

**4.** Preparation according to any one of the preceding claims, **characterized in that** it is substantially free of active oxygen, the term "active oxygen" referring to molecular oxygen as well as to oxygen-containing compounds wherein oxygen has an oxidation state higher than -2, especially peroxides with the general structure R-O-O--R, wherein R and R are selected from the group consisting of alkyl residues and hydrogen, and wherein R and R are the same or different.

**5.** Preparation according to any one of the preceding claims, **characterized in that** it has a mono-layered or multi-layered polymer matrix, with at least one layer having an active substance content.

**6.** Preparation according to claim 5, **characterized in that** the matrix contains one or more polymer(s) selected from the group comprising cellulose ether, especially ethyl cellulose, propyl cellulose, carboxymethyl cellulose (CMC), hydroxypropyl cellulose (HPC), hydroxypropylmethyl cellulose (HPMC), mixtures of cellulose ethers, as well as cellulose acetate, polyvinyl alcohols, polyvinyl acetate, polyvinyl pyrrolidone, polyethylene oxide polymers, polyurethane, polyacrylic acid, polyacrylates, polymethacrylates, alginates, pectins, gelatine, starch and natural rubbers.

**7.** Preparation according to claim 5, **characterized in that** the matrix contains one or more polymer(s) selected from the group of the hydrophile, water-soluble polymers or of the polymers degradable in aqueous media, preferably from the group comprising cellulose derivatives, especially hydroxypropylmethyl cellulose, carboxymethyl cellulose, hydroxypropyl cellulose and methyl cellulose, as well as polyvinyl alcohol, polyvinyl acetate, polyvinyl pyrrolidone, polyacrylates, water-soluble polysaccharides, especially pullulan, xanthan, alginates, dextranes and pectins, proteins, preferably gel-forming proteins, especially gelatine.

8.  Preparation according to any one of the preceding claims, **characterized in that** at least one layer or at least one surface of the preparation has mucoadhesive properties.

9.  Preparation according to any one of the preceding claims, **characterized in that** it contains one or more additives selected from the group of plasticizers, dyes and pigments, degradation enhancers, wetting agent, absorption- or permeation-enhancing substances, pH regulators, fillers, flavouring and aromatic substances and sweeteners.

10. Preparation according to any one of the preceding claims, **characterized in that** it contains at least one active substance which due to its chemical structure is susceptible to attack by peroxide radicals.

11. Use of a preparation according to any one of the preceding claims for the production of a product for transmucosal administration of medicinal active substances, preferably for application in the oral cavity.

12. Use of a preparation according to any one of the preceding claims for the production of a product as an oral administration form for releasing active substances in the gastrointestinal tract.

13. Use of a preparation according to any one of the preceding claims for the production of a product for releasing flavouring or aromatic substances in the oral cavity.

14. Process for the production of a film-like active substance-containing preparation for application in the oral cavity for transmucosal application according to claim 1, **characterized by** the following steps:

    (a) Determining the peroxide number of each and every one of the formulation components which are provided for according to the recipe for making the preparation;
    (b) selecting the formulation components in such a manner that the sum of the peroxide numbers of the individual formulation components is maximally 40, with the peroxide number of each one of the formulation components being weighted according to the percentage of these components in the preparation;
    (c) preparing a solution, dispersion or melt which contains the selected formulation components as well as the active substance(s) to be released;
    (d) coating this solution, dispersion or melt onto an inert support using doctor-knife application, roll application, spraying or extrusion methods, and subsequent drying or cooling, which results in the formation of a film layer.

15. Process according to claim 14, **characterized in that** the sum of the peroxide number is maximally 15, preferably maximally 5.

16. Process according to claim 14 or 15, **characterized in that**, following step (a), at least one formulation component is subjected to a treatment with reducing agent(s) which is/are suitable for reducing the peroxide content.

17. Process according to claim 16, **characterized in that** the mentioned treatment is carried through in such a manner that the aqueous solution of an inorganic sulfite salt or hydrogen sulfite salt is added to the formulation component provided in an alcoholic solution, preferably in methanolic or ethanolic solution.

**Revendications**

1.  Préparation contenant des substances actives sous forme de film pour l'application dans la cavité buccale ou pour l'application à travers la muqueuse avec un indice de peroxyde de 40 au maximum, **caractérisée en ce que** l'indice de peroxyde cité est obtenu en employant au moins un anti-oxydant choisi dans le groupe comprenant le sulfite de sodium, le metabisulfite de sodium, le thioglycérol, l'acide thioglycolique, le gallate de propyle, le gallate d'octyle, le butylhydroxyanisole et la butylhydroxytoluène, à chaque fois en une concentration de 0,001 à 5% en poids.

2.  Préparation selon la revendication 1, **caractérisée en ce qu'**un indice de peroxyde de 15 au maximum est obtenu en employant au moins l'un des anti-oxydants cités en une concentration de 0,01 à 3% en poids.

3.  Préparation selon la revendication 2, **caractérisée en ce qu'**un indice de peroxyde de 5 au maximum est obtenu.

4.  Préparation selon l'une quelconque des revendications précédentes, **caractérisée en ce qu'**elle est essentiellement exempte d'oxygène actif, le terme « oxygène actif » se référant à l'oxygène moléculaire ainsi qu'aux composés oxygénés, dans lesquels l'oxygène présente un degré d'oxydation supérieur à -2, en particulier les peroxydes ayant la structure générale R-O-O-R, dans laquelle R et R sont choisis dans le groupe formé par les radicaux alkyle et l'atome d'hydrogène, et dans laquelle R et R peuvent être identiques ou différents.

**5.** Préparation selon l'une quelconque des revendications précédentes, **caractérisée en ce qu'**elle présente une matrice polymère monocouche ou multicouche, au moins une couche contenant des substances actives.

**6.** Préparation selon la revendication 5, **caractérisée en ce que** la matrice contient un ou plusieurs polymères, qui sont choisis dans le groupe comprenant les éthers de cellulose, en particulier l'éthylcellulose, la propylcellulose, la carboxyméthylcellulose (CMC), l'hydroxypropylcellulose (HPC), l'hydroxypropylméthylcellulose (HPMC), les mélanges d'éthers de cellulose, ainsi que l'acétate de cellulose, les poly(alcool vinylique)s, le poly(acétate de vinyle), les polyvinylpyrrolidones, les polymères poly(oxyde d'éthylène), le polyuréthane, le poly(acide acrylique), les polyacrylates, les polyméthacrylates, les alginates, les pectines, la gélatine, l'amidon et les gommes naturelles.

**7.** Préparation selon la revendication 5, **caractérisée en ce que** la matrice contient un ou plusieurs polymères, qui sont choisis dans le groupe des polymères hydrophiles, hydrosolubles ou susceptibles de se déliter dans les milieux aqueux, de préférence dans le groupe comprenant les dérivés de cellulose, en particulier l'hydroxypropylméthylcellulose, la carboxyméthylcellulose, l'hydroxypropylcellulose et la méthylcellulose, ainsi que le poly(alcool vinylique), le poly(acétate de vinyle), la polyvinylpyrrolidone, les polyacrylates, les polysaccharides hydrosolubles, en particulier le pullulane, le xanthane ; les alginates, les dextranes et les pectines, les protéines, de préférence les protéines gélifiantes, en particulier la gélatine.

**8.** Préparation selon l'une quelconque des revendications précédentes, **caractérisée en ce qu'**au moins une couche ou au moins une surface de la préparation présente des propriétés d'adhérence aux muqueuses.

**9.** Préparation selon l'une quelconque des revendications précédentes, **caractérisée en ce qu'**elle contient un ou plusieurs additifs, choisis dans le groupe formé par les plastifiants, les colorants et pigments, les agents de délitement, les agents mouillants, les substances favorisant la résorption ou la perméation, les régulateurs de pH, les charges, les agents gustatifs et aromatiques et les édulcorants.

**10.** Préparation selon l'une quelconque des revendications précédentes, **caractérisée en ce qu'**elle contient au moins une substance active qui, en raison de sa structure chimique, est réceptive à une attaque par des radicaux peroxyde.

**11.** Utilisation d'une préparation selon l'une quelconque des revendications précédentes pour la préparation d'un produit pour administrer à travers la muqueuse des substances actives médicamenteuses, de préférence pour l'application dans la cavité buccale.

**12.** Utilisation d'une préparation selon l'une quelconque des revendications précédentes pour préparer un produit, sous une forme posologique orale, destiné à libérer des substances actives dans le tractus gastro-intestinal.

**13.** Utilisation d'une préparation selon l'une quelconque des revendications précédentes pour préparer un produit destiné à libérer des substances gustatives ou aromatiques dans la cavité buccale.

**14.** Procédé pour produire une préparation sous forme de film contenant des substances actives, destinée à être appliquée dans la cavité buccale, pour une application à travers la muqueuse selon la revendication 1, **caractérisé par** les étapes opérationnelles suivantes :

(a) détermination de l'indice de peroxyde de chacun des composants prévus selon la formulation pour la production ou préparation;
(b) sélection des composants de formulation de telle façon que la somme des indices de peroxyde des composants individuels de formulation est de 40 au maximum, l'indice de peroxyde de chacun des composants de formulation étant pondéré de façon correspondante à la proportion en pourcentage de ce composant dans la préparation ;
(c) production d'une solution, dispersion ou masse fondue, qui contient les composants de formulation choisis, ainsi que la ou les substances actives à libérer ;
(d) couchage de cette solution, dispersion ou masse fondue sur un substrat inerte au moyen de procédés par raclage, par application au rouleau, par pulvérisation ou extrusion, puis séchage ou refroidissement, formant ainsi une couche de film.

**15.** Procédé selon la revendication 14, **caractérisé en ce que** la somme des indices de peroxyde est de 15 au maximum, de préférence de 5 au maximum.

**16.** Procédé selon la revendication 14 ou 15, **caractérisé en ce qu'**au moins un composant de formulation est soumis à la suite de l'étape (a) à un traitement par un ou des agents réducteurs, lequel ou lesquels conviennent afin de réduire la teneur en peroxyde.

**17.** Procédé selon la revendication 16, **caractérisé en ce que** le traitement cité est réalisé de telle façon que le composant de formulation est mélangé dans une solution alcoolique, de préférence dans une solution méthanolique ou éthanolique, avec la solution aqueuse d'un sel sulfite ou un sel hydrogénosulfite inorganique.

**Fig. 1**

| | Referenz | Stickstoff-Atmosphäre | Na-Bisulfit | Ascorbylpalmitat | Vitamin E |
|---|---|---|---|---|---|
| 2 Wochen | 2,674 | 0,926 | 0,601 | 0,133 | 0,055 |
| 1 Monat | 4,326 | 1,641 | 1,404 | 0,18 | 0,049 |
| 3 Monate | 5,829 | 2,206 | 2,421 | 1,015 | 0,04 |

**EP 1 509 201 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0380367 A

- EP 0539215 A